Europäisches Patentamt

European Patent Office

Office européen des brevets

(19)

(11) Veröffentlichungsnummer: **0 301 450 B1**

(12) **EUROPÄISCHE PATENTSCHRIFT**

(45) Veröffentlichungstag der Patentschrift :
02.05.91 Patentblatt 91/18

(51) Int. Cl.⁵ : **C07C 69/533, C07C 67/38**

(21) Anmeldenummer : **88111912.7**

(22) Anmeldetag : **23.07.88**

(54) **Verfahren zur Herstellung von Pentensäurealkylestern.**

(30) Priorität : 30.07.87 DE 3725241

(43) Veröffentlichungstag der Anmeldung :
01.02.89 Patentblatt 89/05

(45) Bekanntmachung des Hinweises auf die
Patenterteilung :
02.05.91 Patentblatt 91/18

(84) Benannte Vertragsstaaten :
BE CH DE ES FR GB IT LI NL

(56) Entgegenhaltungen :
DE-A- 2 630 086
US-A- 4 303 589
PATENTS ABSTRACTS OF JAPAN, unexamined applications, C Field, Band 6, Nr. 7, 16.
Januar 1982; THE PATENT OFFICE JAPANESE
GOVERNMENT, Seite 14 C 87

(73) Patentinhaber : BASF Aktiengesellschaft
Carl-Bosch-Strasse 38
W-6700 Ludwigshafen (DE)

(72) Erfinder : Maerkl, Robert, Dr.
Bolander Weg 23
W-6701 Fussgoenheim (DE)
Erfinder : Bertleff, Werner, Dr.
Neuzenlache 3
W-6806 Viernheim (DE)
Erfinder : Wilfinger, Hans-Joerg, Dr.
Buschstrasse 11
W-6707 Schifferstadt (DE)
Erfinder : Schuch, Gunter, Dr.
Ulrich-v.Hutten-Strasse 5
W-6700 Ludwigshafen (DE)
Erfinder : Harder, Wolfgang, Dr.
Bergwaldstrasse 16
W-6940 Weinheim (DE)
Erfinder : Kuehn, Gebhard
Am Weidenschlag 92
W-6700 Ludwigshafen (DE)
Erfinder : Panitz, Paul
Wachenheimer Strasse 1
W-6520 Worms 1 (DE)

## Beschreibung

Gegenstand der Erfindung ist ein Verfahren zur Herstellung von Pentensäurealkylestern durch Umsetzen von Butadien mit Kohlenmonoxid und Alkanolen in Gegenwart von Cobaltcarbonylkomplexen und tertiären Stickstoffbasen.

Aus der US-PS 4 550 195 ist ein Verfahren zur Herstellung von Pentensäurealkylestern bekannt, bei dem man Butadien enthaltenden $C_4$-Schnitt mit Kohlenmonoxid und Alkanolen in Gegenwart von Cobaltcarbonylkatalsatoren und heterocyclischen aromatischen tertiären Stickstoffbasen umsetzt. Bei der technischen Durchführung des Verfahrens hat sich herausgestellt, daß die Raum-Zeit-Ausbeute zu wünschen übrig läßt, insbesondere dann, wenn das im Überschuß angewandte Kohlenmonoxid im Kreis geführt und wieder verwendet wird.

Entsprechend Bulletin of the Chem. Soc. of Japan, Band 46 (1973), Seiten 526 und 527 ist auch bereits bekannt, daß man Pentensäuremethylester durch Umsetzung von Butadien mit ca. 26 Vol.% Wasserstoff enthaltendem Kohlenmonoxid und Methanol in Gegenwart von Cobaltcarbonyl und Pyridin erhält. Die Mitverwendung von Wasserstoff wird jedoch nicht als vorteilhaft beurteilt, da erhebliche Mengen an Nebenprodukten anfallen, die Selektivität zu Pentensäuremethylester reduziert wird und die Mitverwendung von Wasserstoff keinen beschleunigenden Effekt auf die Carbalkoxylierung von Butadien hat.

Es war deshalb die technische Aufgabe gestellt, bei der Herstellung von Pentensäurealkylestern durch Carbalkoxylierung von Butadien die Selektivität zu Pentensäurealkylestern und die Raum-Zeit-Ausbeute zu steigern, ohne daß der Anfall an Nebenprodukten erhöht wird.

Diese Aufgabe wird gelöst in einem Verfahren zur Herstellung von Pentensäurealkylestern durch Umsetzen von Butadien mit Kohlenmonoxid und Alkanolen in Gegenwart von Cobaltcarbonylkomplexen und tertiären aromatischen heterocyclischen Stickstoffbasen bei einer Temperatur von 80 bis 160°C und unter einem Druck von 100 bis 1000 bar unter Mitverwendung von Wasserstoff, wobei man 0,1 bis 10 Vol.% Wasserstoff, bezogen auf die Menge an Kohlenmonoxid mitverwendet.

Das neue Verfahren hat den Vorteil, daß es mit hoher Selektivit und hoher Raum-Zeit-Ausbeute verläuft, ohne daß erhöhte Mengen an Nebenprodukten gebildet werden.

Die Mitverwendung von Wasserstoff war insofern nicht angezeigt, als entsprechend Bulltetin of the Chem. Soc. of Japan, Band 46 (1973), Seite 526 keine Beschleunigung der Carbalkoxylierung von Butadien zu erwarten war und zudem mit einer erhöhten Menge an Nebenprodukten zu rechnen war.

Als Ausgangsverbindung verwendet man Butadien. Geeignet sind auch Butadien enthaltende Kohlenwasserstoffgemische, insbesondere Butadien enthaltende $C_4$-Schnitte. Solche $C_4$-Schnitte enthalten, z.B. durchschnittlich 40 bis 60 Gew.% Butadien, 20 bis 35 Gew.% Isobuten, 10 bis 25 Gew.% Buten-1, 2 bis 15 Gew.% Buten-2, 1 bis 10 Gew.% Butan.

Geeignete Alkanole haben vorteilhaft 1 bis 6 Kohlenstoffatome, insbesondere 1 bis 4 Kohlenstoffatome. Beispielhaft seien genannt: Methanol, Ethanol, Isopropanol, Butanol oder Hexanol. Besonders bevorzugt ist Methanol.

Die Alkanole wendet man in der Regel im Überschuß an. Vorteilhaft verwendet man je Mol Butadien 1,1 bis 10 Mol, insbesondere 1,1 bis 5 Mol Alkanole.

Die Umsetzung wird bei einer Temperatur von 80 bis 160°C, insbesondere 100 bis 150°C durchgeführt. Ferner hält man bei der Umsetzung einen Druck von 100 bis 1000 bar, insbesondere von 120 bis 700 bar ein.

Kohlenmonoxid wird vorteilhaft im Überschuß, z.B. dem 1,3- bis 10-fachen der stöchiometrisch erforderlichen Menge angewandt. Sofern das Verfahren kontinuierlich durchgeführt wird, führt man Kohlenmonoxid fortwährend im Kreis und ergänzt es mit frischem Kohlenmonoxid.

Die verwendeten Cobaltcarbonylkatalysatoren werden entweder in situ aus Cobaltsalzen, z.B. fettsauren Cobaltsalzen wie Cobaltformiat, -acetat, -propionat oder -butyrat erzeugt. Vorteilhaft bringt man den Katalysator bereits als Cobaltcarbonyl ein. Insbesondere hat es sich bewährt, wenn man Cobaltcarbonylkatalysator gelöst im Butadien oder $C_4$-Schnitt in das Reaktionsgemisch einbringt. Eine solche Lösung erhält man beispielsweise durch Umsetzen einer wäßrigen Lösung von fettsauren Cobaltsalzen mit einem Gemisch aus Kohlenmonoxid und Wasserstoff in Gegenwart von Aktivkohle bei einer Temperatur von 100 bis 160°C und unter einem Druck von 100 bis 400 bar. Aus der wäßrigen Lösung wird das entstandene Cobaltcarbonyl dann mit der olefinisch ungesättigten Verbindung extrahiert.

Die Umsetzung wird in Gegenwart von heterocyclischen, aromatischen tertiären Stickstoffbasen vorteilhaft mit einem $pK_a$-Wert von 6 bis 9 durchgeführt. Geeignete Stickstoffbasen sind beispielsweise 3-Methylpyridin ($pK_a$ 6,0), 4-Methylpyridin ($pK_a$ 6,0), 2,3-Dimethylpyridin ($pK_a$ 6,6), 2,4-Dimethylpyridin ($pK_a$ 7,0) oder 3,5-Dimethylpyridin ($pK_a$ 6,2). Besonders technische Bedeutung haben 3-Methylpyridin und 4-Methylpyridin erlangt. Es ist auch möglich, Gemische der genannten Stickstoffbasen anzuwenden. Besonders bewährt hat es sich, wenn man je Mol Cobaltcarbonylkatalysator 2 bis 25 Mol der vorgenannten Stickstoffbase anwendet.

Je Mol Butadien verwendet man vorteilhaft 0,01 bis 0,25 Mol Cobaltkatalysator, insbesondere 0,04 bis 0,2 Mol Cobaltkatalysator.

Erfindungsgemäß werden, bezogen auf die angewandte Menge an Kohlenmonoxid 0,1 bis 10 Vol.%, insbesondere 0,3 bis 8 Vol.% Wasserstoff mitverwendet. In der Regel werden Kohlenmonoxid und Wasserstoff gemeinsam der Reaktion zugeführt. Es ist jedoch auch möglich, Kohlenmonoxid und Wasserstoff getrennt zuzugeben. Vorteilhaft wird die Umsetzung kontinuierlich, z.B. in mehreren hintereinander geschalteten Stufen, z.B. 2 bis 4 Stufen, insbesondere Schlaufenreaktoren durchgeführt. Eine besondere Ausführungsform hierbei ist es, den Wasserstoff, z.B. in der ersten und/oder nachfolgenden Stufen, z.B. der zweiten Stufe, zuzugeben.

Das Verfahren nach der Erfindung führt man beispielsweise durch, indem man zwei hintereinander geschaltete Hochdruckgefäße aus nicht-rostendem Stahl, z.B. zwei hintereinander geschaltete Schlaufenreaktoren verwendet und in die erste Stufe fortlaufend Butadien enthaltenden $C_4$-Schnitt, Alkanole, Cobaltcarbonylkatalysatoren, heterocyclische, aromatische tertiäre Stickstoffbasen, Kohlenmonoxid und Wasserstoff in den angegebenen Verhältnissen zuführt und das Reaktionsgemisch auf die angegebene Temperatur und den angegebenen Druck hält. In dem Maße wie Ausgangsstoffe zugeführt werden, wird in der zweiten Stufe Reaktionsgemisch entnommen und Kohlenmonoxid und überschüssiger Wasserstoff abgetrennt und wiederum mit frischem Kohlenmonoxid und Wasserstoff dem Ausgangsgemisch zugesetzt. Das verbleibende Reaktionsgemisch wird nach Entspannung und Abtrennen des Cobaltkatalysators, z.B. durch Behandeln in wäßrigsaurem Medium mit molekularen Sauerstoff enthaltenden Gasen, Abtrennen der Cobaltsalzlösung und Destillation der organischen Phase aufgearbeitet.

Nach dem Verfahren der Erfindung erhält man vornehmlich 3-Pentensäureester mit wechselnden Mengen an 4- und 2-Pentensäureestern, die sich zur Herstellung von Adipinsäureestern eignen.

Das Verfahren nach der Erfindung sei an folgenden Beispielen veranschaulicht. Die angegebenen Gewichtsteile verhalten sich zu den Volumenteilen wie Kilogramm zu Liter.

Beispiel 1

Einem Hochdruckgefäß von 260 Vol.-Teilen führt man von unten im Verlauf von 60 Minuten 33 Gew.-Teile $C_4$-Schnitt mit 41% (m/m) Butadien-1,3, 28 Gew.-Teile 3-Methylpyridin, 11 Gew.-Teile Methanol, 1 Gew.-Teil Cobalt in Form von Cobaltcarbonyl und 27 Gew.-Teile eines Gasgemisches folgender Zusammensetzung 82 Vol.% Kohlenmonoxid, 1 Vol.% Kohlendioxid, 3 Vol.% Stickstoff und 2 Vol.% Wasserstoff sowie 11 Vol.% Butene zu. Die Carbonylierung verläuft bei einer Temperatur von 135°C und unter einem Druck von 650 bar. Das am Kopf des Hochdruckgefäßes abgenommene Produkt wird entspannt und das abgetrennte Gas wiederum in die Synthese zurückgeführt. Anschließend werden die überschüssigen $C_4$-Kohlenwasserstoffe abdestilliert. Sie enthalten noch 1500 ppm nicht umgesetztes Butadien. Der Umsatz bezogen auf Butadien beträgt 99,78%, die Selektivität zu Pentensäuremethylester beträgt 92%. Der Anteil an Nebenprodukten beträgt 7,1%. Die Raumzeitausbeute beträgt 0,1 kg Pentensäuremethylester je Liter Reaktionsraum und Stunde.

Vergleichsbeispiel

Man verfährt wie in Beispiel 1 beschrieben. Dem Reaktionsgefäß werden stündlich 26,4 Gew.-Teile $C_4$-Schnitt mit 41% (m/m) Butadien-1,3, 22,4 Gew.-Teile 3-Methylpyridin, 8,8 Gew.-Teile Methanol, 0,8 Gew.-Teile Cobalt in Form von Cobaltcarbonyl und 21,6 Gew.-Teile eines Gasgemisches folgender Zusammensetzung, 83 Vol.% Kohlenmonoxid, 1 Vol.% Kohlendioxid, 3 Vol.% Stickstoff, 0,07 Vol.% Wasserstoff sowie 12 Vol.% Butene zugeführt. Die Umsetzung erfolgt wie in Beispiel 1 beschrieben. Der Umsatz bezogen auf Butadien beträgt 99,8%, die Selektivität zu Pentensäuremethylester beträgt 92%. Der Anteil an Nebenprodukten beträgt 7,1%. Die Raumzeitausbeute beträgt 0,081 kg Pentensäuremethylester je Liter Reaktionsraum und Stunde.

## Ansprüche

1. Verfahren zur Herstellung von Pentensäurealkylestern durch Umsetzung von Butadien mit Kohlenmonoxid und Alkanolen in Gegenwart von Cobaltcarbonylkomplexen und tertiären, aromatischen heterocyclischen Stickstoffbasen bei einer Temperatur von 80 bis 160°C und unter einem Druck von 100 bis 1000 bar unter Mitverwendung von Wasserstoff, dadurch gekennzeichnet, daß man 0,1 bis 10 Vol.% Wasserstoff, bezogen auf die angewandte Menge Kohlenmonoxid mitverwendet.

2. Verfahren nach Anspruch 1, dadurch gekennzeichnet, daß man Kohlenmonoxid im Kreis führt.

3. Verfahren nach den Ansprüchen 1 und 2, dadurch gekennzeichnet, daß man eine Temperatur von 100 bis 150°C einhält.

4. Verfahren nach den Ansprüchen 1 bis 3, dadurch gekennzeichnet, daß man einen Druck von 120 bis 700 bar einhält.

5. Verfahren nach den Ansprüchen 1 bis 4, dadurch gekennzeichnet, daß man 3-Methylpyridin oder 4-Methylpyridin oder deren Gemische verwendet.

6. Verfahren nach den Ansprüchen 1 bis 5, dadurch gekennzeichnet, daß man Butadien enthaltenden $C_4$-Schnitt verwendet.

## Claims

1. A process for preparing an alkyl pentenoate by reacting butadiene with carbon monoxide and an alkanol in the presence of a cobalt carbonyl complex and a tertiary aromatic heterocyclic nitrogen base at from 80 to 160°C and at from 100 to 1,000 bar in the presence of from 0.1 to 10% by volume of hydrogen, based on the amount of carbon monoxide used.

2. A process as claimed in claim 1, wherein carbon monoxide is recycled.

3. A process as claimed in either of claims 1 and 2, wherein a temperature of from 100 to 150°C is maintained.

4. A process as claimed in any of claims 1 to 3, wherein a pressure of from 120 to 700 bar is maintained.

5. A process as claimed in any of claims 1 to 4, wherein 3-methylpyridine or 4-methylpyridine or a mixture thereof is used.

6. A process as claimed in any of claims 1 to 5, wherein a butadiene-containing $C_4$ cut is used.

## Revendications

1. Procédé de préparation d'esters alkyliques d'acide ; penténiques par réaction de butadiène avec du monoxyde de carbone et des alcanols en présence de complexes cobalt-carbonyle et de bases azotées hétérocycliques aromatiques tertiaires à une température de 80 à 160°C et sous une pression de 100 à 1000 bars avec utilisation simultanée d'hydrogène, caractérisé en ce qu'on utilise simultanément de 0,1 à 10% en volume d'hydrogène par rapport à la quantité utilisée de monoxyde de carbone.

2. Procédé selon la revendication 1, caractérisé en ce qu'on fait passer en circuit fermé monoxyde de carbone.

3. Procédé selon la revendication 1 ou 2, caractérisé en ce qu'on maintient une température de 100 à 150°C.

4. Procédé selon l'une quelconque des revendications 1 à 3, caractérisé en ce qu'on maintient une pression de 120 à 700 bars.

5. Procédé selon l'une quelconque des revendications 1 à 4, caractérisé en ce qu'on utilise de la 3-méthylpyridine, de la 4-méthylpyridine ou des mélanges de celles-ci.

6. Procédé selon l'une quelconque des revendications 1 à 5, caractérisé en ce qu'on utilise une coupe en $C_4$ contenant du butadiène.